(19) Europäisches Patentamt
European Patent Office
Office européen des brevets

(11) Numéro de publication: 0 049 674
B1

# (12) FASCICULE DE BREVET EUROPEEN

(45) Date de publication du fascicule du brevet: **14.11.84**

(51) Int. Cl.³: **C 07 C 69/24**, C 07 C 67/38, C 07 C 69/44

(21) Numéro de dépôt: **81420139.8**

(22) Date de dépôt: **28.09.81**

(54) **Procédé de préparation d'esters par carbonylation de composés monooléfiniques.**

(30) Priorité: **03.10.80 FR 8021542**

(43) Date de publication de la demande: **14.04.82 Bulletin 82/15**

(45) Mention de la délivrance du brevet: **14.11.84 Bulletin 84/46**

(84) Etats contractants désignés: **AT BE CH DE FR GB IT LI LU NL SE**

(56) Documents cités:
**US - A - 3 397 226**
**US - A - 3 668 249**

(73) Titulaire: **RHONE-POULENC CHIMIE DE BASE, 25, quai Paul Doumer, F-92408 Courbevoie (FR)**

(72) Inventeur: **Jenck, Jean, 2, rue Lakanal, F-69100 Villeurbanne (FR)**

(74) Mandataire: **Varniere-Grange, Monique et al, RHONE-POULENC RECHERCHES Service Brevets Chimie et Polymères Centre de Recherches de Saint-Fons B.P. 62, F-69192 St-Fons Cédex (FR)**

EP 0 049 674 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation d'esters par carbonylation de composés monooléfiniques, c'est-à-dire par réaction du monoxyde de carbone et d'un alcool sur des composés de formule $R_1CH = CHR_2$ dans laquelle:

$R_1$ et $R_2$, identiques ou différents, représentent l'hydrogène ou un radical alkyle ayant au maximum 20 atomes de carbone, pouvant être substitué par 1 ou 2 atomes de chlore ou groupes alcoxy renfermant au maximum 4 atomes de carbone,

$R_1$ pouvant en outre représenter un radical $-(CH_2)_p-COOH$, $-(CH_2)_p-COOR_3$ ou $-(CH_2)_p-CN$ dans lequel p est un entier au plus égal à 6 pouvant être nul, $R_3$ représente un radical alkyle comportant au maximum 12 atomes de carbone, un à deux groupements méthylène pouvant en outre comporter un substituant alkyle ayant au maximum 4 atomes de carbone,

$R_1$ et $R_2$ pouvant en outre former ensemble un radical unique bivalent $-(CH_2)_q-$, comportant le cas échéant un ou deux substituants alkyle ayant au maximum 4 atomes de carbone, q étant un entier compris entre 3 et 6 inclus.

L'invention a plus spécifiquement pour objet, la préparation de diesters à partir de penténoates d'alkyles. Un objet particulier de la présente invention est la synthèse d'adipates d'alkyles par carbonylation des pentène-3 oates d'alkyles.

Il est bien connu, d'après le «Bulletin of the Chemical Society of Japan, vol. 46, 1973, pages 526 et 527», qu'on obtient un mélange renfermant des diesters d'alkyles et notamment, un adipate d'alkyle, en faisant réagir du monoxyde de carbone et un alcool sur un pentène-3 oate d'alkyle, sous haute pression et à température élevée, en présence de cobalt carbonyle et d'une base azotée hétérocyclique et aromatique. Cependant le développement à l'échelle industrielle d'une telle technique, dont l'intérêt de principe n'est pas contesté, est largement compris par la faible activité du système catalytique mis en oeuvre.

Il a maintenant été trouvé qu'il est possible de préparer des esters par carbonylation de composés monooléfiniques avec une efficacité accrue, en faisant réagir du monoxyde de carbone et un alcool sur un composé renfermant une liaison oléfinique unique, en présence de cobalt, d'une base azotée tertiaire choisie parmi les hétérocycles azotés formés de 5 à 6 chainons, pouvant comporter un ou deux substituants choisis parmi les groupes alkyles ou alcoxy ayant au maximum 4 atomes de carbone, le groupe hydroxy et les atomes d'halogène, renfermant éventuellement 2 ou 3 doubles liaisons, et pouvant, par ailleurs, le cas échéant, être soudés à un noyau benzénique, sous réserve que les maillons adjacents à l'hétéroatome d'azote ne soient ni substitués, ni communs à deux cycles, dont le $pK_a$ est compris entre 3 et 10 et de ruthénium.

Selon le présennt procédé on fait donc réagir du monoxyde de carbone et un alcool R-OH sur un composé de formule $R_1CH = CHR_2$, dans laquelle $R_1$ et $R_2$ ont la signification précédemment donnée, et R est un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle.

Les matières de départ susceptibles d'être carbonylées selon le présent procédé sont donc des composés renfermant une liaison oléfinique unique interne ou terminale; ces composés comportent plus spécifiquement de 3 à 20 atomes de carbone.

Par la mise en oeuvre du présent procédé on obtient des esters saturés, c'est-à-dire des composés qui renferment d'une part un groupement carboxylate (-COOR) et, d'autre part un atome d'hydrogène de plus que la matière de départ. Parmi ces esters prédomine le composé dont le groupement carboxylate (-COOR) est situé en position terminale sur la chaîne principale de la matière de départ; dans la suite du présent mémoire, on désignera par «esters linéaires» ce type particulier de composés.

Une première catégorie de matières de départ plus particulièrement adaptées a pour formule:

$R_1CH = CHR_2$ dans laquelle $R_1$ et $R_2$, identiques ou différents représentent l'hydrogène ou un radical alkyle ayant au maximum 10 atomes de carbone, ou bien forment ensemble un radical unique bivalent $-(CH_2)_q-$ q ayant la signification précédente, ledit radical pouvant comporter le cas échéant 1 ou 2 substituants méthyle. A titre d'exemples de tels composés on peut citer le propylène, le butène-1, le butène-2, les hexènes, les octènes et le dodécène-1.

Une seconde catégorie de matières de départ plus particulièrement appropriées est constituée par les composés de formule :

$R_1CH = CHR_2$ dans laquelle $R_1$ représente un radical $-(CH_2)_p-COOR_3$, p et $R_3$ ayant la signification précédente, un à deux groupements méthylène pouvant comporter un substituant alkyle ayant au maximum 4 atomes de carbone, et

$R_2$ représente l'hydrogène ou un radical alkyle ayant au maximum 4 atomes de carbone.

Parmi les composés de ce type les penténoates d'alkyles revêtent un intérêt tout particulier, car ils permettent d'accéder aux adipates d'alkyles, intermédiaires de l'acide adipique.

Le présent procédé nécessite la mise en oeuvre d'un alcool de formule ROH, R ayant la signification donnée précédemment.

A titre d'exemples d'alcools utilisables dans le cadre du présent procédé on peut citer le méthanol, l'éthanol, l'isopropanol, le n-propanol, le tertio butanol, le n-hexanol, le cyclohexanol, l'éthyl-2 hexanol-1, le dodécanol-1, l'éthylène glycol, l'hexanediol-1,6, l'alcool benzylique, l'alcool phényléthylique et le phénol.

On utilise de préférence un alcanol ayant au maximum 4 atomes de carbone; le méthanol et l'éthanol conviennent bien à la mise en oeuvre du présent procédé.

On peut engager l'alcool et le composé monooléfinique en quantités stoechiométriques. Cependant on utilise de préférence un excès d'alcool, dans la proportion de 1 à 10, ou encore mieux, de 2 à 5 moles d'alcool par mole de composé monooléfinique.

La réaction est conduite en présence de cobalt.

N'importe quelle source de cobalt susceptible de réagir avec le monoxyde de carbone dans le milieu réactionnel pour donner in-situ des complexes carbonyles du cobalt peut être utilisée dans le cadre du présent procédé.

Les sources typiques de cobalt sont par exemple le cobalt métallique finement divisé, des sels inorganiques tels que le nitrate ou le carbonate de cobalt, des sels organiques en particulier des carboxylates. Peuvent également être employés les cobalt-carbonyles ou hydrocarbonyles; le dicobaltoctacarbonyle convient bien à la mise en oeuvre du présent procédé.

Le rapport molaire entre le composé monooléfinique et le cobalt est en général compris entre 10 et 1000. Ce rapport est avantageusement fixé à une valeur comprise entre 20 et 300.

Le procédé selon la présente invention nécessite également la présence d'une base azotée tertiaire choisie parmi les hétérocycles azotés formés de 5 à 6 chainons, pouvant comporter un ou deux substituants choisis parmi les groupes alkyles ou alcoxy ayant au maximum 4 atomes de carbone, le groupe hydroxy et les atomes d'halogène, renfermant éventuellement 2 ou 3 doubles liaisons, et pouvant, par ailleurs, les cas échéant être soudés à un noyau benzénique, sous réserve que les maillons adjacents à l'hétéroatome d'azote ne soient ni substitués, ni communs à deux cycles et dont le $pK_a$ est compris entre 3 et 10.

Conviennent plus particulièrement à la mise en oeuvre du présent procédé les hétérocycles azotés à 6 chainons, de $pK_a$ compris entre 4 et 7, notamment la pyridine, la picoline-4, l'isoquinoléine et la lutidine-3,5.

La quantité de base azotée tertiaire utilisée est en général telle que le rapport molaire N/Co soit compris entre 1 et 50. Pour une bonne mise en oeuvre de l'invention la demanderesse préconise de fixer ce rapport à une valeur comprise entre 4 et 25.

L'une des caractéristiques essentielles du présent procédé réside dans l'adjonction de ruthénium au système catalytique à base de cobaltet d'une amine tertiaire. La forme précise, sous laquelle le ruthénium est engagé à la réaction n'est pas fondamentale. Conviennent particulièrement à la mise en oeuvre de l'invention le triruthéniumdodécarbonyle et, plus généralement, tout composé du ruthénium susceptible de conduire dans les conditions réactionnelles à l'apparition in-situ de ruthénium carbonyles. A ce titre on peut citer notamment le ruthénium métallique sous forme finement divisée, les carboxylates de ruthénium (en particulier l'acétate de ruthénium) les halogénures de ruthénium (en particulier le trichlorure de ruthénium) et l'acétylacétonate de ruthénium.

La quantité de ruthénium à mettre en oeuvre n'est pas critique. La teneur en ruthénium du milieu réactionnel, ayant une influence favorable sur la vitesse de réaction, sera déterminée, notamment, en fonction de la vitesse que l'on estimera convenable d'atteindre. De manière générale la présence d'une quantité aussi faible que 0,005 atome-gramme de ruthénium par atome-gramme de cobalt conduit à des résultats appréciables. Il ne s'avère pas souhaitable d'engager plus de 5 atomes-grammes de ruthénium par atome-gramme de cobalt. De bons résultats sont obtenus lorsque le rapport atomique Ru/Co est compris entre 0,01 et 2,5, ou encore mieux entre 0,1 et 1 environ.

Selon la présente invention on fait donc réagir du monoxyde de carbone et un alcool (ROH) sur un composé monooléfinique en présence du système catalytique défini ci-avant. La réaction est conduite en phase liquide à température supérieure à 120°C, sans qu'il soit utile de dépasser 200°C, sous une pression de monoxyde de carbone d'au moins 50 bars et pouvant atteindre 1000 bars. On préfère opérer à une température de l'ordre de 130 à 180°C et sous une pression de monoxyde de carbone de l'ordre de 100 à 300 bars.

Bien entendu, les conditions de pression et de température optimales seront d'autant plus sévères que la matière de départ sera moins réactive, ce qui se produit, notamment, lorsque le degré de protection stérique de la double liaison augmente.

On peut faire usage de solvants ou de diluants inertes dans les conditions réactionnelles. Des solvants de ce type sont, par exemple, des hydrocarbures aliphatiques ou aromatiques tels que le pentane, le cyclohexane, le benzène et le toluène. Toutefois, les produits du procédé ou les alcools de départ sont des solvants ou diluants tout à fait appropriés.

On utilisé du monoxyde de carbone sensiblement pur, tel qu'il se présente dans le commerce. Toutefois, la présence d'impuretés telles que du dioxyde de carbone, du méthane ou de l'azote n'est pas nuisible; la présence de traces d'hydrogène (inférieures à 3% en volume) tend à stabiliser le système catalytique.

Comme cela a été indiqué en tête du présent mémoire, le procédé selon la présente invention trouve une application plus particulièrement intéressante dans la synthèse de diesters à partir de penténoates d'alkyles. En général, on met en oeuvre un pentène-3 oate d'alkyle, bien que les pentène-2 oates, les pentène-4 oates et des mélanges de penténoates d'alkyles puissent être utilisés. Dans le cadre de cette application, il s'avère préférable de choisir l'alcool (coréactif) correspondant au reste alkyle de l'ester de départ, le reste alkyle ayant avantageusement 4 atomes de carbone du maximum. De bons résultats sont obtenus au départ de l'un ou l'autre des couples de réactifs suivant: penténoate de méthyle et méthanol, penténoate d'éthyle et éthanol.

Lorsqu'on travaille avec une forte concentration de cobalt, c'est-à-dire avec un rapport molaire du penténoate d'alkyle au cobalt situé dans la gamme allant de 10 à 50, environ, pour obtenir une forte proportion de diester linéaire (adipate) il s'avère préférable de limiter le rapport atomique Ru/Co à une valeur comprise entre 0,01 et 0,25, le rapport molaire N/Co étant fixé à une valeur comprise entre 3 et 6 environ.

Lorsqu'on travaille avec une faible concentration de cobalt, c'est-à-dire avec un rapport molaire du composé monooléfinique au cobalt situé entre 150 et 350 environ, pour obtenir une forte proportion d'adipate il s'avère préférable de fixer le rapport atomique Ru/Co à une valeur comprise entre 0,25 et 1, environ, et d'augmenter le rapport molaire N/Coà une valeur située dans la gamme allant de 8 à 25 environ.

Dans ce mode de réalisation, qui constitue une variante avantageuse du présent procédé, la présence de traces d'hydrogène de l'ordre de 1 % au maximum du volume du monoxyde de carbone a un effet particulièrement favorable sur le déroulement de la réaction.

Les exemples ci-après illustrent l'invention sans toutefois en limiter le domaine ou l'esprit.

*Exemples*

Les conventions suivantes sont utilisées ci-après.

Dans les produits formés ne sont pas inclus les composés résultant de l'isomérisation de position de la double liaison oléfinique.

=/CO désigne le rapport molaire du composé monooléfinique (matière de départ) au cobalt.
TT (%) désigne le nombre de moles de produits formés pour 100 moles de matière de départ chargées.
RT (%) désigne le nombre de moles d'esters pour 100 moles de produits formés.
S (%) désigne le nombre de moles d'ester linéaire pour 100 moles d'esters formés, communément taux de linéarité.
$Ru(acac)_3$ désigne l'acétylacétonate de ruthénium.
$Ru(OAc)_3$ désigne l'acétate de ruthénium.
T (°C) désigne la température en degrés celsius.
t désigne la durée d'un essai à la température indiquée, exprimée en heures.
A désigne l'activité exprimée en moles de produits formés par heure et par atomegramme de cobalt.
X (%) désigne le nombre de moles de diesters pour 100 moles de produits formés.
Y (%) désigne le nombre de moles d'adipate d'alkyle pour 100 moles de produits formés.
Z (%) désigne le nombre de moles de pentanoate d'alkyle pour 100 moles de produits formés.

*Exemples 1 à 6*

Dans le tableau I ci-après on a rassemblé les conditions particulières ainsi que les résultats obtenus lors d'une série d'essais de carbonylation de l'hexène-1. Le mode opératoire utilisé est le suivant.

Dans un autoclave de 125 $cm^3$ en acier inoxydable purgé sous courant d'argon, on introduit de l'hexène-1, du dicobaltoctacarbonyle, du triruthéniumdodécacarbonyle, du méthanol et de l'isoquinoléine (sauf mention contraire). L'autoclave est alors purgé par un courant de monoxyde de carbone, renfermant 0,7% en volume d'hydrogène, puis on le porte à la température indiquée dans le tableau I ci-après, sous une pression de 130 bars.

Après deux heures de réaction, à cette température, l'autoclave est refroidi et dégazée. Le mélange réactionnel est alors analysé par chromatographie en phase gazeuse.

Les essais témoins a, b et c figurant également dans le tableau I, sont réalisés en l'absence de ruthénium.

*Exemples 7 à 12*

Dans le tableau II ci-après on a rassemblé les conditions particulières ainsi que les résultats obtenus lors d'une seconde série d'essais réalisés sur divers composés monooléfiniques et selon le mode opératoire décrit ci-avant, le cobalt étant engagé sous forme de dicobaltoctacarbonyle sauf mention contraire.

L'essai témoin d figurant également dans le tableau II ci-après est réalisé en l'absence de ruthénium.

TABLEAU I

| Exemple n° | hexène-1 (m.Mol) | méthanol (m.Mol) | cobalt (m.At-g) | =/Co | N/Co | Ru/Co | T (°C) | TT (%) | RT (%) | S (%) |
|---|---|---|---|---|---|---|---|---|---|---|
| a(*) | 103 | 500 | 4,0 | 26 | 21 | 0 | 150 | 23 | ND | 82 |
| 1(*) | 100 | 500 | 4,0 | 25 | 22 | 0,5 | 150 | 61 | ND | 81 |
| 2(**) | 100 | 502 | 4,0 | 25 | 22 | 0,1 | 150 | 57 | 100 | 76 |
| b | 101 | 303 | 1,92 | 53 | 4,3 | 0 | 130 | 27 | 100 | 83 |
| 3 | 100 | 301 | 1,90 | 53 | 4,2 | 0,1 | 130 | 42 | 100 | 86 |
| 4 | 100 | 300 | 2,00 | 50 | 4,1 | 0,33 | 130 | 39 | 100 | 86 |
| 5 | 101 | 304 | 1,90 | 53 | 4,3 | 1,05 | 130 | 36 | 100 | 86 |
| c | 80 | 200 | 2,80 | 28 | 4,2 | 0 | 160 | 18 | 98,7 | 83 |
| 6 | 66 | 198 | 2,60 | 25 | 4,2 | 0,25 | 160 | 48 | 96,0 | 75 |

(*) essai réalisé avec de la pyridine, sous 150 bars. pendant 1 heure.
(**) essai réalisé avec de l'éthanol, de la pyridine, sous 150 bars, pendant 1 heure.
ND : non déterminé.

TABLEAU II

| Ex. n° | substrat (m.Mol) | alcool (m.Mol) | cobalt (m.At-g) | base | ruthénium | =/CO | N/Co | Ru/Co | P (bars) | T t | TT (%) | RT (%) | S (%) |
|---|---|---|---|---|---|---|---|---|---|---|---|---|---|
| d(*) | hexène--3 83,3 | métha-nol 154 | 2 | picoline--4 | — | 42 | 4,0 | 0 | 130 | 135°C 2 h. | 0 | — | — |
| 7(*) | hexène--3 101 | métha-nol 150 | 2 | picoline--4 | $Ru(CO)_{12}$ | 51 | 3,9 | 2,3 | 130 | 135°C 2 h. | 75 | 100 | 83 |
| 8 | tétradé-cène-1 100 | métha-nol 501 | 0,96 | lutidine--3,5 | $Ru(acac)_3$ | 104 | 8,4 | 2 | 130 | 160°C 16 h. | 48 | 98,6 | 68 |
| 9 | cyclo-hexène 100 | éthanol 298 | 1,98 | isoqui-noléine | $RuCl_3$ | 50,5 | 12,1 | 0,25 | 200 | 190°C 2 h. | 67 | 99,3 | 100 |
| 10 | dodé-cène-1 100 | éthanol 499 | 4,0 | pyridine | $RuCl_3$ | 25 | 22 | 0,50 | 150 | 150°C 1 h. | 23 | 100 | 80 |
| 11 | hexène--2 100 | éthanol 498 | 4,0 | pyridine | $Ru(acac)_3$ | 25 | 22 | 0,50 | 150 | 150°C 1 h. | 11 | 100 | 69 |
| 12(**) | pentène--3 ni-trile 100 | métha-nol 148 | 2,04 | picoline--4 | $Ru_3(CO)_{12}$ | 49 | 4,0 | 0,15 | 130 | 160°C 4 h. | 44 | 78,3 | 78 |

(*) essai réalisé avec du carbonate de cobalt, dans 10 $cm^3$ de toluène
(**) essai réalisé dans 10 $cm^3$ de propionate d'éthyle.

*Exemples 13 à 16*

Dans un autoclave de 125 $cm^3$ en acier inoxydable purgé sous courant d'argon, on introduit 100 mMol d'alcool (dont la nature est précisée dans le tableau III ci-après), 50 mMol du pentène-3 oate d'alkyle correspondant, 1 mMol de dicobaltoctacarbonyle, 8 mMol d'isoquinoléine (le rapport molaire N/Co est donc égal à 4) et un composé du ruthénium dont la nature et la quantité introduite sont indiquées également dans le tableau III ci-après.

On purge alors l'autoclave par un courant de monoxyde de carbone, renfermant 0,7% (en volume) d'hydrogène, puis on le porte à 160°C sous une pression de 130 bars. Les résultats obtenus en une heure de réaction à 160°C sont portés dans le tableau précité.

Les essais témoins e et f sont réalisés en l'absence de ruthénium.

TABLEAU III

| Ex. n° | Alcool | Ruthénium Nature | m.Mol | Ru/Co | A | X (%) | Y (%) | Z (%) |
|---|---|---|---|---|---|---|---|---|
| e | éthanol | — | 0 | 0 | 5,6 | 93 | 73,5 | 7 |
| 13 | éthanol | $Ru_3(CO)_{12}$ | 0,066 | 0,10 | 12 | 87,8 | 70,0 | 10,3 |
| f | méthanol | — | 0 | 0 | 6,8 | 93,2 | 78,0 | 6,8 |
| 14 | méthanol | $Ru_3(CO)_{12}$ | 0,022 | 0,035 | 15,1 | 93,1 | 77,0 | 6,4 |
| 15 | méthanol | $Ru(acac)_3$ | 0,2 | 0,10 | 15,8 | 92,0 | 76,7 | 7,5 |
| 16 | méthanol | $Ru(OAc)_3$ | 0,4 | 0,10 | 14,5 | 90,7 | 75,7 | 8,2 |

Ce tableau montre que l'adjonction d'une faible quantité de ruthénium permet de multiplier la vitesse de réaction, sans modifier de manière sensible la répartition des produits obtenus.

*Exemples 17 à 24*

On réalise une série d'essais selon le mode opératoire décrit précédemment en faisant réagir du monoxyde de carbone à 0,7% (en volume) d'hydrogène sur une charge renfermant 100 mMol de pentène-3 oate de méthyle, 200 mMol de méthanol, du dicobaltoctacarbonyle, du triruthéniumdodécarbonyle et, sauf mention contraire de l'isoquinoléine. Les conditions particulières ainsi que les résultats obtenus en deux heures de réaction à 160°C sous une pression de 130 bars sont protés dans le tableau IV ci-après.

Les essais témoins g et h, figurant également dans ce tableau sont réalisés en l'absence de ruthénium.

*Exemples 25 à 40*

On réalise une série d'essais selon le mode opératoire décrit précédemment en faisant réagir du monoxyde de carbone à 0,7% (en volume) d'hydrogène

sur une charge renfermant 100 mMol de pentène-3 oate de méthyle, 200 mMol de méthanol, du dicobaltoctacarbonyle, du ruthénium chargé sous forme de divers composés dont la nature est indiquée dans le tableau V ci-après, et de l'isoquinoléine. Les conditions particulières ainsi que les résultats obtenus en deux heures de réaction à 160°C sous une pression de 130 bars sont portés dans le tableau V ci-après.

Les essais témoins i, j et k, figurant également dans ce tableau sont réalisés en l'absence de ruthénium.

Du tableau V il ressort d'une part qu'en l'absence de ruthénium, l'augmentation du rapport molaire N/Co entraîne une diminution très nette de la vitesse de réaction et, d'autre part, que l'adjonction de ruthénium permet d'obtenir une vitesse de réaction accrue, la proportion de diesters étant pratiquement inchangée et qu'une augmentation du rapport molaire Ru/Co entraîne une diminution sensible de la proportion d'adipate. Toutefois, on constate dans ce dernier cas qu'une augmentation du rapport molaire N/Co permet d'obtenir une proportion élevée d'adipate avec une vitesse appréciable.

TABLEAU IV

| Ex. n° | $Co_2(CO)_8$ (m.Mol) | Isoquinoléine (m.Mol) | $Ru_3(CO)_{12}$ (m.Mol) | =/Co | N/Co | Ru/Co | A | X(%) | Y(%) | Z(%) |
|---|---|---|---|---|---|---|---|---|---|---|
| g | 0,77 | 6,0 | 0 | 65 | 3,9 | 0 | 6,7 | 93,8 | 79,7 | 5,5 |
| 17 | 0,74 | 6,3 | 0,05 | 67 | 4,3 | 0,1 | 19,2 | 90,6 | 74,6 | 8,3 |
| 18 | 0,79 | 6,9 | 0,13 | 63 | 4,4 | 0,25 | 18,2 | 92,1 | 75,4 | 6,8 |
| 19 | 0,78 | 6,1 | 0,37 | 64 | 3,9 | 0,70 | 21,3 | 86,5 | 67,0 | 12,1 |
| 20 | 0,76 | 6,6 | 0,50 | 66 | 4,3 | 1,00 | 22,6 | 86,2 | 66,7 | 12,1 |
| h | 0,68 | 11,0 | 0 | 74 | 8,1 | 0 | 5,1 | 93,6 | 80,5 | 5,8 |
| 21 | 075 | 12,1 | 0,50 | 67 | 8,1 | 1,00 | 18,8 | 86,6 | 69,3 | 12,6 |
| 22 | 0,73 | 17,5 | 0,50 | 69 | 12 | 1,03 | 13,1 | 85,3 | 68,3 | 13,8 |
| 23(*) | 0,71 | 17,2 | 0,50 | 70 | 12 | 1,06 | 19,4 | 87,4 | 71,4 | 11,6 |
| 24(*) | 0,72 | 28,8 | 0,12 | 69 | 20 | 0,25 | 11,1 | 92,0 | 77,5 | 7,4 |

(*) essai réalisé avec de la pyridine (en remplacement de l'isoquinoléine).

TABLEAU V

| Ex. n° | $CO_2(CO)_8$ m.Mol | Ruthénium Nature | =/Co | N/Co | Ru/CO | A | X(%) | Y(%) | Z(%) |
|---|---|---|---|---|---|---|---|---|---|
| i | 0,20 | — | 245 | 4,8 | 0 | 8,45 | 90 | 69 | 7,5 |
| j | 0,225 | — | 227 | 8,1 | 0 | 8,40 | 91,8 | 75 | 6,6 |
| k | 0,205 | — | 237 | 20,3 | 0 | 1,7 | 90,5 | 71,5 | 9,5 |
| 25 | 0,205 | $Ru(acac)_3$ | 242 | 4,9 | 0,25 | 10,1 | 91,5 | 72,9 | 3,5 |
| 26 | 0,21 | $RuCl_3.nH_2O$ | 235 | 4,8 | 0,24 | 20,1 | 86,6 | 64,5 | 9,3 |
| 27 | 0,185 | $Ru_3(CO)_{12}$ | 265 | 4,5 | 0,27 | 15,4 | 88,7 | 62,2 | 7,7 |
| 28 | 0,21 | » | 240 | 8,7 | 0,24 | 18,9 | 92,2 | 72,8 | 6,3 |
| 29 | 0,195 | » | 261 | 21,7 | 0,26 | 23,7 | 94,1 | 78,8 | 5,2 |
| 30 | 0,23 | $Ru(acac)_3$ | 213 | 4,3 | 0,86 | 17,1 | 88,2 | 64,8 | 3,2 |
| 31 | 0,225 | $Ru_3(CO)_{12}$ | 221 | 4,5 | 0,90 | 21,9 | 86,4 | 57,7 | 10 |
| 32 | 0,21 | » | 238 | 5,7 | 0,96 | 23,2 | 89,6 | 63,3 | 7,8 |
| 33 | 0,205 | » | 238 | 9 | 0,97 | 28,6 | 90,5 | 67,5 | 7,4 |
| 34 | 0,21 | » | 236 | 12 | 0,95 | 31,3 | 91,7 | 72,6 | 7,1 |
| 35 | 0,21 | » | 240 | 19,4 | 0,96 | 33,3 | 91,8 | 75 | 7,2 |
| 36 | 0,195 | » | 258 | 42,5 | 1,04 | 12,9 | 89,3 | 71,7 | 9,1 |
| 37 | 0,22 | » | 228 | 4,1 | 2,5 | 24,2 | 87,5 | 54 | 8,8 |
| 38 | 0,195 | » | 253 | 20,9 | 2,6 | 32,7 | 86,4 | 65,7 | 12,5 |
| 39 | 0,215 | » | 224 | 40,0 | 2,4 | 14,7 | 88,1 | 65,9 | 8,1 |
| 40 | 0,21 | $Ru(acac)_3$ | 233 | 5,6 | 4,7 | 18,8 | 81,1 | 48,4 | 16,9 |

*Exemple 41*

Dans l'appareillage et selon le mode opératoire décrit précédemment on fait réagir du monoxyde de carbone exempt d'hydrogène sur une charge constitué par:

100 mMol de pentène-3 oate de méthyle,
200 mMol de méthanol
2 mMol de dicobaltoctacarbonyle,
24 mMol d'isoquinoléine et
0,33 mMol de triruthéniumdodécacarbonyle.

Les résultats obtenus en deux heures de réaction à 160°C sous une pression totale de 80 bars sont les suivants:

A = 6,1
X = 87,1%
Y = 73,4%
Z = 12%

*Exemple 42*

Dans l'appareillage et selon le mode opératoire décrits précédemment on fait réagir du monoxyde de carbone renfermant environ 0,35% (en volume) d'hydrogène sur une charge constituée par:

100 mMol de pentène-3 oate de méthyle,
500 mMol de méthanol,
1 mMol de dicobaltoctacarbonyle,
8 mMol de picoline-4 et
0,22 mMolde triruthéniumdodécacarbonyle.

Les résultats obtenus en deux heures de réactionà 140°C sous une pression totale de 250 bars sont les suivants:

A = 10,3
X = 95,5%
Y = 76,0%
Z = 3,5%

*Exemple 43*

Dans l'appareillage et selon le mode opératoire décrits précédemment on fait réagir du monoxyde de carbone renfermant environ 0,35% (en volume) d'hydrogène sur une charge constituée par:

200 mMol de pentène-3 oate de méthyle,
400 mMol de méthanol,
2 mMol de dicobaltoctacarbonyle,
24 mMol d'isoquinoléine
0,33 mMol de triruthéniumdodécacarbonyle.

Les résultats obtenus en deux heures de réaction à 180°C sous une pression totale de 80 bars sont les suivants:

A = 7,2
X = 56,6%
Y = 39%
Z = 41%

*Essai témoin 1:*

On reproduit l'exemple 43 en l'absence de ruthénium. On n'observe pratiquement pas de réaction.

*Exemples 44 et 45*

Dans l'appareillage et selon le mode opératoire décrits précédemment on réalise une série d'essais en faisant réagir du monoxyde de carbone renfermant des traces d'hydrogène, dont le pourcentage en volume est désigné par $H_2$ (%) dans le tableau VI ci-après, sur une charge renfermant 100 mMol de pentène-3 oate de méthyle, 200 mMol de méthanol, du dicobaltoctacarbonyle,, du triruthéniumdodécacarbonyle et de l'isoquinoléine.

Les conditions particulières ainsi que les résultats obtenus en deux heures de réaction à 160°C sous une pression totale de 130 bars sont portés dans le tableau VI ci-après.

Les essais témoins m à p figurant également dans ce tableau sont réalisés en l'absence de ruthénium.

Dans ledit tableau on a également fait figurer les exemples 31 et 35 ainsi que l'essai témoin k.

Ce tableau confirme l'intérêt de l'adjonction de ruthénium au milieu réactionnel dans lequel le cobalt se trouve en une concentration relativement faible, y compris en l'absence d'hydrogène.

TABLEAU VI

| Ex. n° | $Co_2(CO)_8$ (m.Mol) | Isoquinoléine (m.Mol) | =/Co | $H_2$ (%) | N/Co | Ru/Co | A | X (%) | Y (%) | Z (%) |
|---|---|---|---|---|---|---|---|---|---|---|
| m | 0,22 | 1,80 | 227 | 0 | 4,1 | 0 | ng | ND | ND | ND |
| 44 | 0,22 | 1,80 | 227 | 0 | 4,1 | 1,0 | 19,2 | 93,9 | 72,5 | 6,1 |
| n | 0,22 | 8,80 | 227 | 0 | 20 | 0 | ng | ND | ND | ND |
| 45 | 0,22 | 8,80 | 227 | 0 | 20 | 1,0 | 10,0 | 90,6 | 69,6 | 9,0 |
| p | 0,18 | 1,90 | 278 | 0,7 | 5,2 | 0 | 8,4 | 89,3 | 68,3 | 3,4 |
| 31 | 0,22 | 1,98 | 221 | 0,7 | 4,5 | 0,9 | 21,9 | 86,4 | 57,5 | 10 |
| 35 | 0,21 | 8,15 | 240 | 0,7 | 19,4 | 0,96 | 33,3 | 91,8 | 75 | 7,2 |
| k | 0,21 | 8,5 | 237 | 0,7 | 20,3 | 0 | 1,7 | 90,5 | 71,5 | 9,5 |

ng: négligeable
ND: non déterminé

*Exemples 46 à 48*

On réalise une série d'essais selon le mode opératoire décrit ci-avant en faisant réagir du monoxyde de carbone renfermant 0,8% (en volume) d'hydrogène sur une charge renfermant 50 mMol de pentène-3 oate de méthyle, du méthanol, du dicobaltoctacarbonyle, du rutheniumdodécacarbonyle, de l'isoquinoléine et 10 $cm^3$ de benzène. Les conditions particulières ainsi que les résultats obtenus en deux heures de réaction à 160°C sous une pression de 130 bars sont portés dans le tableau VII ci-après.

TABLEAU VII

| Exemple n° | $CO_2(CO)_8$ m.Mol | Méthanol m.Mol | N/Co | Ru/Co | TT (%) | X (%) | Y (%) | Z (%) |
|---|---|---|---|---|---|---|---|---|
| 46 | 0,93 | 98 | 4,2 | 0,21 | 65,0 | 93,3 | 79,9 | 5,5 |
| 47 | 0,95 | 43 | 4,2 | 0,21 | 44,7 | 93,6 | 78,0 | 5,9 |
| 48 | 2,04 | 100 | 3,9 | 0,10 | 51,6 | 94,2 | 79,6 | 5,8 |

**Revendications**

1. Procédé de préparation d'esters saturés par réaction du monoxyde de carbone et d'un alcool de formule ROH dans laquelle R est un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle, sur un composé monooléfinique de formule

$$R_1CH = CHR_2$$

dans laquelle $R_1$ et $R_2$, identiques ou différents, représentent l'hydrogène ou un radical alkyle ayant au maximum 20 atomes de carbone, pouvant être substitué par 1 ou 2 atomes de chlore ou groupes alcoxy renfermant au maximum 4 atomes de carbone,

$R_1$ pouvant en outre représenter un radical $-(CH_2)_2-COOH$, $-(CH_2)_p-COOR_3$ ou $-(CH_2)_p-CN$ dans lequel p est un entier au plus égal à 6 pouvant être nul, $R_3$ représente un radical alkyle comportant au maximum 12 atomes de carbone, un à deux groupements méthylène pouvant en outre comporter un substituant alkyle ayant au maximum 4 atomes de carbone,

$R_1$ et $R_2$ pouvant en outre former ensemble un radical unique bivalent $-(CH_2)_q-$, comportant le cas échéant un ou deux substituants alkyle ayant au maximum 4 atomes de carbone, q étant un entier compris entre 3 et 6 inclus, en présence de cobalt, d'une base azotée tertiaire choisie parmie les héterocycles azotés formés de 5 à 6 chainons, pouvant comporter un ou deux substituants choisis parmi les groupes alkyles ou alcoxy au maximum 4 atomes de carbone, le groupe hydroxy et les atomes d'halogène, renfermant éventuellement 2 ou 3 doubles liaisons, et pouvant, par ailleurs, le cas échéant, être soudés à un noyau benzénique, sous réserve que les maillons adjacents à l'hétéroatome d'azote ne soient ni substitués, ni communs à deux cycles, dont le $pK_a$ est compris entre 3 et 10 et de ruthénium, les dits esters renfermant un groupe carboxylate (-COOR) et un atome d'hydrogène de plus que le composé monooléfinique.

2. Procédé selon la revendication 1, caractérisé en ce que le composé monooléfinique renferme de 3 à 20 atomes de carbone.

3. Procédé selon l'une quelconque des revendications 1 ou 2, caractérisé en ce que le composé monooléfinique a pour formule:

$$R_1CH = CHR_2$$

dans laquelle $R_1$ et $R_2$, identiques ou différents représentent l'hydrogène ou un radical alkyle ayant au maximum 10 atomes de carbone, ou bien forment ensemble un radical unique bivalent $-(CH_2)_q-$ q ayant la signification donnée dans la revendication 2, ledit radical pouvant comporter le cas échéant 1 ou 2 substituants méthyle.

4. Procédé selon l'une quelconque des revendications 1 ou 2, caractérisé en ce que le composé monooléfinique a pour formule:

$$R_1CH = CHR_2$$

dans laquelle $R_1$ représente un radical $-(CH_2)_p-COOR_3$ p et $R_3$ ayant la signification donnée dans la revendication 1, un à deux groupements méthylène pouvant comporter un substituant alkyle ayant au maximum 4 atomes de carbone, et $R_2$ représente l'hydrogène ou un radical alkyle ayant au maximum 4 atomes de carbone.

5. Procédé selon la revendication 4, caractérisé en ce que le composé monooléfinique est un penténoate d'alkyle.

6. Procédé selon l'une quelconque des revendications précédentes, caractérisé en ce que l'alcool est un alcanol ayant au maximum 4 atomes de carbone.

7. Procédé selon la revendication 1, caractérisé en ce que la base azotée tertiaire est choisie parmi les hétérocycles azotés à 6 chainons et de $pK_a$ compris entre 4 et 7.

8. Procédé selon l'une quelconque des revendications précédentes, caractérisé en ce que le rapport molaire entre le composé monooléfinique et le cobalt est compris entre 10 et 1000 et, de préférence entre 20 et 300.

9. Procédé selon l'une quelconque des revendications précédentes, caractérisé en ce que le rapport molaire entre l'alcool et le composé monooléfinique est compris entre 1 et 10 et, de préférence entre 2 et 5.

10. Procédé selon l'une quelconque des revendications précédentes, caractérisé en ce que le rapport molaire N/Co est compris entre 1 et 50 et, de préférence entre 4 et 25.

11. Procédé selon l'une quelconque des revendications précédentes, caractérisé en ce que le rapport atomique Ru/Co est compris entre 0,005 et 5 et, de préférence entre 0,01 et 2,5.

12. Procédé selon la revendication 11, caractérisé en ce que le rapport atomique Ru/Co est compris entre 0,10 et 1 environ.

13. Procédé selon l'une quelconque des revendications précédentes, caractérisé en ce que la réaction est conduite en phase liquide à une température comprise entre 120 et 200°C sous une pression de monoxyde de carbone comprise entre 50 et 1000 bars.

14. Procédé selon la revendication 13, caractérisé en ce que la réaction est conduite à une température comprise entre 130 et 180°C, sous une pression de monoxyde de carbone comprise entre 100 et 300 bars, environ.

15. Procédé selon l'une quelconque des revendications 5 à 14, caractérisé en ce que le rapport molaire du penténoate d'alkyle au cobalt est compris entre 10 et 50 environ, le rapport atomique Ru/Co est compris entre 0,01 et 0,25 et le rapport molaire N/Co est compris entre 3 et 6 environ.

16. Procédé selon l'une quelconque des revendications 5 à 14, caractérisé en ce que le rapport molaire du penténoate d'alkyle au cobalt est compris entre 150 et 350 environ, le rapport atomique Ru/Co est compris entre 0,25 et 1, environ et le rapport molaire N/Co est compris entre 8 et 25 environ.

17. Procédé selon l'une quelconque des revendications précédentes, caractérisé en ce que le monoxyde de carbone renferme des traces d'hydrogène n'excédant pas 1% (en volume).

**Patentansprüche**

1. Verfahren zur Herstellung von gesättigten Estern durch Einwirken-lassen von Kohlenmonoxid und eines Alkohols der Formel ROH, in der R eine gegebenenfalls durch ein oder zwei Hydroxylgruppen substituierte Alkylgruppe mit maximal 12 Kohlenstoffatomen, eine Cycloalkylgruppe mit 5 bis 7 Kohlenstoffatomen, eine Aralkylgruppe mit 7 bis 12 Kohlenstoffatomen oder eine Phenylgruppe ist, auf eine monoolefinische Verbindung der Formel

$$R_1CH = CHR_2,$$

in der $R_1$ und $R_2$, gleich oder verschieden, (jeweils) für Wasserstoff oder eine Alkylgruppe mit maximal 20 Kohlenstoffatomen, die mit ein oder zwei Chloratomen oder Alkoxygruppen mit maximal 4 Kohlenstoffatomen substituiert sein kann, stehen, wobei

$R_1$ weiterhin eine Gruppe $-(CH_2)_p-COOH$, $-(CH_2)_p-COOR_3$ oder $-(CH_2)_p-CN$, in der p eine ganze Zahl von höchstens 6 oder auch Null ist (und) $R_3$ eine Alkylgruppe mit maximal 12 Kohlenstoffatomen (oder) ein oder zwei Methylengruppen, die darüber hinaus einen Alkylsubstituenten mit maximal 4 Kohlenstoffatomen haben können, sein können, $R_1$ und $R_2$ weiterhin zusammen eine einzige 2-wertige Gruppe $-(CH_2)_q-$ bilden können, die gegebenenfalls ein oder zwei Alkylsubstituenten mit maximal 4 Kohlenstoffatomen aufweist, wobei q eine ganze Zahl von 3 bis 6 einschliesslich ist, in Gegenwart von Cobalt (und) einer tertiären Stickstoffbase, ausgewählt unter den 5- oder 6gliedrigen stickstoffhaltigen Heterocyclen, die ein oder zwei Substituenten, ausgewählt unter den Alkylgruppen oder Alkoxygruppen mit maximal 4 Kohlenstoffatomen, Hydroxygruppe und Halogenatome aufweisen können, gegebenenfalls zwei oder drei Doppelbindungen aufweisen und ausserdem gegebenenfalls mit einem Benzolring kondensiert sein können, mit der Massgabe, dass die dem Heteroatom Stickstoff benachbarten Ringatome weder substituiert noch beiden Ringen gemeinsam sind, deren $pK_a$ 3 bis 10 beträgt, und von Ruthenium, wobei die Ester eine Carboxylatgruppe (-COOR) und ein Wasserstoffatom mehr als die Monoolefinverbindung aufweisen.

2. Verfahren nach Anspruch 1, dadurch gekennzeichnet, dass die Monoolefinverbindung 3 bis 20 Kohlenstoffatome enthält.

3. Verfahren nach Anspruch 1 oder 2, dadurch gekennzeichnet, dass die monoolefinische Verbindung der Formel

$$R_1CH = CHR_2$$

entspricht, in der $R_1$ und $R_2$, gleich oder verschieden, Wasserstoff oder eine Alkylgruppe mit maximal 10 Kohlenstoffatomen bedeuten oder zusammen eine einzige 2wertige Gruppe $-(CH_2)_q-$ bilden, in der q die in Anspruch 2 gegebene Bedeutung hat, wobei diese Gruppe gegebenenfalls ein oder zwei Methylsubstituenten enthalten kann.

4. Verfahren nach Anspruch 1 oder 2, dadurch gekennzeichnet, dass die monoolefinische Verbindung der Formel

$$R_1CH = CHR_2$$

entspricht, in der $R_1$ eine Gruppe $-(CH_2)_p-COOR_3$, in der p und $R_3$ die in Anspruch 1 gegebene Bedeutung haben, ein oder zwei Methylengruppen, die einen Alkylsubstituenten mit maximal 4 Kohlenstoffatomen aufweisen können, bedeutet und $R_2$ Wasserstoff oder eine Alkylgruppe mit maximal 4 Kohlenstoffatomen ist.

5. Verfahren nach Anspruch 4, dadurch gekennzeichnet, dass die monoolefinische Verbindung ein Alkylpentenoat ist.

6. Verfahren nach einem der vorangehenden Ansprüche, dadurch gekennzeichnet, dass der Alkohol ein Alkanol mit maximal 4 Kohlenstoffatomen ist.

7. Verfahren nach Anspruch 1, dadurch gekennzeichnet, dass die tertiäre Stickstoffbasis unter den 6gliedrigen stickstoffhaltigen Heterocyclen mit einem $pK_a$-Wert von 4 bis 7 ausgewählt wird.

8. Verfahren nach einem der vorangehenden Ansprüche, dadurch gekennzeichnet, dass das Molverhältnis von monoolefinischer Verbindung zu Cobalt 10 bis 1000 und vorzugsweise 20 bis 300 beträgt.

9. Verfahren nach einem der vorangehenden Ansprüche, dadurch gekennzeichnet, dass das Molverhältnis von Alkohol zu monoolefinischer Verbindung 1 bis 10 und vorzugsweise 2 bis 5 beträgt.

10. Verfahren nach einem der vorangehenden Ansprüche, dadurch gekennzeichnet, dass das Molverhältnis N/Co 1 bis 50 oder vorzugsweise 4 bis 25 beträgt.

11. Verfahren nach einem der vorangehenden Ansprüche, dadurch gekennzeichnet, dass das Atomverhältnis Ru/Co 0,005 bis 5 und vorzugsweise 0,01 bis 2,5 beträgt.

12. Verfahren nach Anspruch 11, dadurch gekennzeichnet, dass das Atomverhältnis Ru/Co zwischen etwa 0,10 und 1 liegt.

13. Verfahren nach einem der vorangehenden Ansprüche, dadurch gekennzeichnet, dass die Umesterung in flüssiger Phase bei einer Temperatur von 120 bis 200°C unter einem Kohlenmonoxiddruck von 50 bis 1000 bar ausgeführt wird.

14. Verfahren nach Anspruch 13, dadurch gekennzeichnet, dass die Reaktion bei einer Temperatur von 130 bis 180°C unter einem Kohlenmonoxiddruck von 100 bis 300 bar etwa ausgeführt wird.

15. Verfahren nach einem der Ansprüche 5 bis 14, dadurch gekennzeichnet, dass das Molverhältnis von Alkylpentenoat zu Cobalt 10 bis 50 etwa, das Atomverhältnis Ru/Co 0,01 bis 0,25 und das Molverhältnis N/Co 3 bis 6 etwa beträgt.

16. Verfahren nach einem der Ansprüche 5 bis 14, dadurch gekennzeichnet, dass das Molverhältnis von Alkylpentenoat zu Cobalt 150 bis 350 etwa, das Atomverhältnis Ru/Co 0,25 bis 1 etwa und das Molverhältnis N/Co 8 bis 25 etwa beträgt.

17. Verfahren nach einem der vorangehenden Ansprüche, dadurch gekennzeichnet, dass das Kohlenmonoxid Spuren von Wasserstoff enthält, die nicht mehr als 1 Vol.-% ausmachen.

**Claims**

1. Process for the preparation of saturated esters by the reaction of carbon monoxide and alcohol of the formula ROH in which R is an alkyl radical containing up to 12 carbon atoms optionally substituted with one or two hydroxy groups, a cycloalkyl radical containing from 5 to 7 carbon atoms, an aralkyl radical containing from 7 to 12 carbon atoms, or a phenyl radical, with a monoolefinic compound of the formula

$$R_1CH = CHR_2$$

in which $R_1$ and $R_2$, which are identical or different, denote hydrogen or an alkyl radical containing up to 20 carbon atoms, which may be substituted with 1 or 2 chlorine atoms or alkoxy groups containing up to 4 carbon atoms,

$R_1$ being moreover capable of denoting a $-(CH_2)_p-COOH$, $-(CH_2)_p-COOR_3$ or $-(CH_2)_p-CN$ radical in which p is an integer from 0 to 6, $R_3$ denotes an alkyl radical containing up to 12 carbon atoms, one or two methylene groups being moreover capable of having an alkyl substituent containing up to 4 carbon atoms,

$R_1$ and $R_2$ being moreover capable to together forming a single divalent $-(CH_2)_q-$ radical, containing, where appropriate, one or two alkyl substituents containing up to 4 carbon atoms, q being an integer from 3 to 6 inclusive, in the presence of cobalt, a tertiary nitrogenous base chosen from the nitrogen-based heterocyclic rings having 5 to 6 members, being capable of having one or two substituents chosen from the alkyl or alkoxy groups containing up to 4 carbon atoms, the hydroxy group or halogen atoms, optionally containing 2 or 3 double bonds, and being capable, moreover, where appropriate, of being joined to a benzene nucleus, provided that the ring members adjacent to the nitrogen heteroatom are neither substituted nor common to two rings, the $pK_a$ of which is between 3 and 10, and ruthenium, the said esters containing a carboxylate group (-COOR) and one hydrogen atom more than the monoolefinic compound.

2. Process according to Claim 1, characterised in that the monoolefinic compound contains from 3 to 20 carbon atoms.

3. Process according to either of Claims 1 or ^, characterised in that the monoolefinic compound has the formula

$$R_1CH = CHR_2$$

in which $R_1$ and $R_2$, which are identical or different, denote hydrogen or an alkyl radical containing up to 10 carbon atoms, or together form a single divalent radical $-(CH_2)_q-$, q having the meaning given in Claim 2, the said radical being capable, where appropriate, of having 1 or 2 methyl substituents.

4. Process according to either of Claims 1 or 2, characterised in that the monoolefinic compound has the formula

$$R_1CH = CHR_2$$

in which $R_1$ denotes à $-(CH_2)_p-COOR_3$ radical, p and $R_3$ having the meaning given in Claim 1, one or two methylene groups being capable of having an alkyl substituent containing up to 4 carbon atoms, and $R_2$ denotes hydrogen or an alkyl radical containing up to 4 carbon atoms.

5. Process according to Claim 4, characterised in that the monoolefinic compound is an alkyl pentenoate.

6. Process according to any one of the preceding claims, characterised in that the alcohol is an alkanol containing up to 4 carbon atoms.

7. Process according to Claim 1, characterised in that the tertiary nitrogenous base is chosen from 6-membered nitrogen-based heterocyclic compounds with a $pK_a$ value of between 4 and 7.

8. Process according to any one of the preceding claims, characterised in that the molar ratio of the monoolefinic compound to cobalt is between 10 and 1,000, and preferably between 20 and 300.

9. Process according to any one of the preceding claims, characterised in that the molar ratio of the alcohol to the monoolefinic compound is between 1 and 10, and preferably between 2 and 5.

10. Process according to any one of the preceding claims, characterised in that the molar ratio N/Co is between 1 and 50, and preferably between 4 and 25.

11. Process according to any one of the preceding claims, characterised in that the atomic ratio Ru/Co is between 0.005 and 5, and preferably between 0.01 and 2.5.

12. Process according to Claim 11, characterised in that the atomic ratio Ru/Co is between 0.10 and 1 approximately.

13. Process according to any one of the preceding claims, characterised in that the reaction is carried out in a liquid phase at a temperature between 120 and 200°C under a carbon monoxide pressure of between 50 and 1,000 bars.

14. Process according to Claim 13, characterised in that the reaction is carried out at a temperature between 130 and 180°C, under a carbon monoxide pressure of between 100 and 300 bars, approximately.

15. Process according to any one of Claims 5 to 14, characterised in that the molar ratio of the alkyl pentenoate to cobalt is between 10 and 50 approximately, the atomic ratio Ru/Co is between 0.01 and 0.25, and the molar ratio N/Co is between 3 and 6 approximately.

16. Process according to any one of Claims 5 to 14, characterised in that the molar ratio of the alkyl pentenoate to cobalt is between 150 and 350 approximately, the atomic ratio Ru/Co is between 0.25 and 1, approximately, and the molar ratio N/Co is between 8 and 25 approximately.

17. Process according to any one of the preceding claims, characterised in that the carbon monoxide contains traces of hydrogen not exceeding 1% (by volume).